# EUROPEAN PATENT SPECIFICATION

(11) **EP 2 773 621 B1**
(45) Date of publication and mention of the grant of the patent: **30.12.2015**
(21) Application number: 12780174.4
(22) Date of filing: 26.10.2012
(51) Int. Cl.: C07D 223/16

(54) **A PROCESS FOR MAKING CRYSTALLINE DELTA-FORM OF IVABRADINE HYDROCHLORIDE**
VERFAHREN ZUR HERSTELLUNG EINER KRISTALLINEN DELTA-FORM VON IVABRADINHYDROCHLORID
PROCÉDÉ DE PRÉPARATION DE LA FORME CRISTALLINE DELTA DU CHLORHYDRATE DE L'IVABRADINE

(30) Priority: 04.11.2011 WO PCT/EP2011/069431
(43) Date of publication of application: 10.09.2014
(73) Proprietor: Synthon BV, 6545 CM Nijmegen (NL)
(72) Inventor: WESTHEIM, Raymond Jozef Hubertus, 6545 CM Nijmegen (NL)
(74) Representative: Mendivil Gil, Maria Dolores
(86) International application number: PCT/EP2012/071264
(87) International publication number: WO 2013/064427

(56) References cited:
- US-A1- 2007 082 885
- US-A1- 2007 082 886
- US-A1- 2010 179 316

## Description

The present invention relates to an improved process for making the pharmaceutically advantageous crystalline delta-form of ivabradine hydrochloride.

### BACKGROUND OF THE INVENTION

Ivabradine , chemically 3-[3-({[(7*S*)-3,4-dimethoxybicyclo[4.2.0]octa-1,3,5-trien-7-yl]methyl}(methyl)amino)propyl]-7,8-dimethoxy-2,3,4,5-tetrahydro-1H-3-benzazepin-2-one of formula (I) 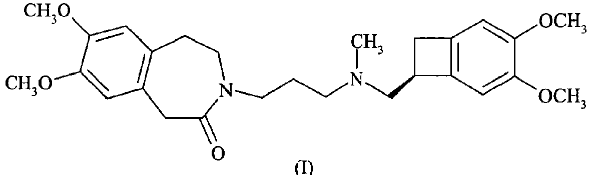 is a pharmaceutically active substance, which is used for the symptomatic management of stable angina pectoris. It reduces the heart rate by a mechanism different from beta-blockers and calcium channel blockers, which are commonly prescribed antianginal drugs.

The molecule of the compound (I) has one chiral carbon atom in position 7. Ivabradine is the single (S) enantiomer.

In the marketed products, which are film-coated tablets for oral administration and are sold, e.g., under trade name Procolan by Servier, ivabradine is present as a hydrochloride.

Ivabradine hydrochloride was first disclosed in EP 534859 (US 5296482). A crystalline ivabradine hydrochloride was obtained therein by treatment of ivabradine base with 0.1N HCl and recrystallization of the residual mass after evaporation of the mixture from acetonitrile in 55% yield.

Various crystalline forms (polymorphs) of ivabradine hydrochloride, which are characterized by a certain distinct pattern of signals in XRPD spectrum, are known in the art.

Alfa- crystalline form has been disclosed in US 7176197 and is obtainable by a crystallization of ivabradine hydrochloride from a toluene/1-methyl-2-pyrrolidone mixture.

Beta- crystalline form was disclosed in US 2006/0194962 and is obtainable by crystallization of ivabradine hydrochloride from water or an isopropanol-water mixture. This form is a hydrate and, if heated, it may be converted to an anhydrate beta-d crystalline form as shown in US 2006/0194965.

Gamma- crystalline form was disclosed in US 2006/0194963 and is obtainable by a crystallization of ivabradine hydrochloride from 2-ethoxtethanol, a mixture of 2-ethoxyethanol and water or a mixture of ethanol and water. This form is a hydrate and, if heated, it may be converted to an anhydrate gamma-d crystalline form as shown in US 2006/0194964.

The EP 1775288 (US appl. 2007/0082885) discloses so called delta-form of crystalline ivabradine hydrochloride. The product was prepared by a crystallization of the product of EP 534859 from acetonitrile and isolation of the crystalline product from the reaction mixture after 2 days standing by filtration and drying at ambient temperature and humidity conditions. According to the analysis, the crystalline form delta is a hydrate comprising about 2.8% of water.

EP 1775287 (US appl. 2007/0082886) discloses so called delta-d form of crystalline ivabradine hydrochloride. The product was prepared by a crystallization of the product of EP 534859 from acetonitrile and isolation of the crystalline product from the reaction mixture after 2 days standing by filtration and heating of the solid product at 85°C for 4 hours. According to the analysis, the crystalline form delta-d is an anhydrate product.

The delta-form of crystals of ivabradine hydrochloride has good handling properties for pharmaceutical applications and thus it was considered by the present inventor as an advantageous crystalline form.

An internal analysis performed by the present inventor, which will be discussed in more detail below, revealed that the anhydrate delta-d form is metastable at ambient conditions and may be converted to the hydrated delta-form under prolonged storing at enhanced aerial humidity. Similarly, the hydrated delta- form may be converted to the anhydrated delta-d form upon heating at low humidity or upon drying *in vacuo.* In further it was confirmed that, based on conditions of crystallization and drying, mixtures of the isomorphic delta and delta-d forms may be formed by the prior art procedures.

Thus, it is an objective need of finding an improved crystallization process which would lead to reliable formation of the stable hydrated delta- form of ivabradine hydrochloride, advantageously with less potential of contamination with other forms. Advantageously, such process would not employ toxic acetonitrile as the crystallization solvent.

### BRIEF DESCRIPTION OF THE PRESENT INVENTION

The present invention relates to a novel process of making the delta-form of crystalline ivabradine hydrochloride essentially free from other crystalline forms thereof, which is simple, rapid and reliable.

In the first aspect, the invention relates to a process of making the delta-form of crystalline ivabradine hydrochloride comprising crystallization of ivabradine hydrochloride from a mixture of an aliphatic alcohol of 1-4 carbon atoms and an aliphatic/alicyclic hydrocarbon of 5-8 carbon atoms. In a preferred aspect, the alcohol is ethanol and the hydrocarbon is n-heptane.

In a particular aspect, the invention relates to a process of making the delta-form of crystalline ivabradine hydrochloride comprising
a) Providing a solution of ivabradine hydrochloride in an aliphatic alcohol of 1-4 carbon atoms, preferably at a temperature of at least 50°C
b) Adding aliphatic/alicyclic hydrocarbon of 5-8 carbon atoms to the obtained solution
c) Optionally, seeding the obtained mixture with seed crystals of the delta-form of crystalline ivabradine hydrochloride
d) Stirring the obtained suspension
e) Filtration of the solid and drying the solid at room temperature.

### BRIEF DESCRIPTION OF THE DRAWINGS

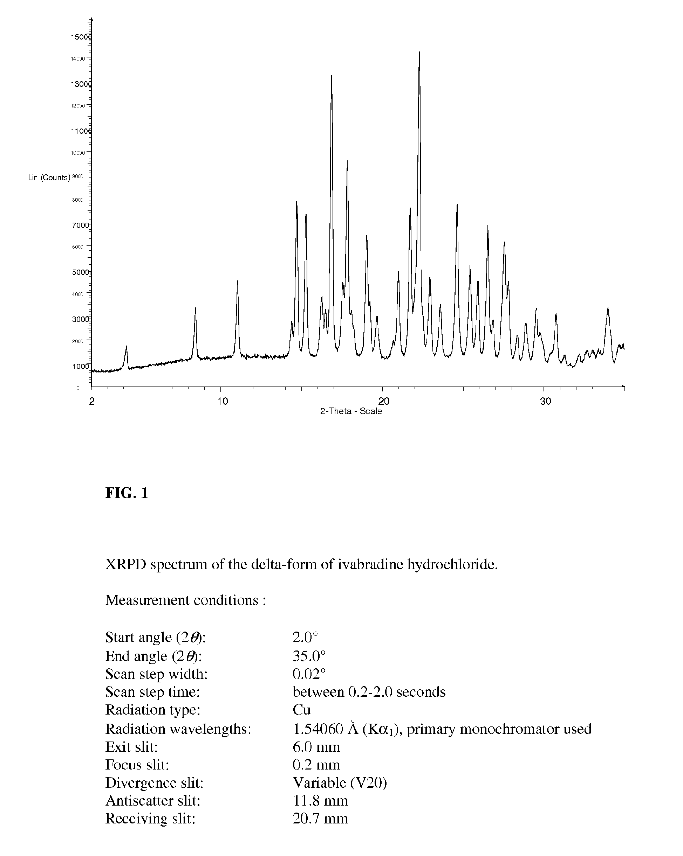

Figure 1 shows the full XRPD pattern of the delta-form of ivabradine hydrochloride.

### DETAILED DESCRIPTION OF THE INVENTION

As discussed above, many polymorphic forms of crystalline ivabradine hydrochloride are known in the art. The hydrated delta-form of ivabradine hydrochloride is relatively stable at ambient conditions of storage and has good handling properties in pharmaceutical applications. The present invention is a result of a thorough study of properties of this crystalline form and methods for making it.

For clarity, the "delta-form" of ivabradine hydrochloride is the polymorph, which has been characterized in US 2007/0082885 by XRPD pattern having the characteristic signals at angles 2 theta as shown in the table:

| Line no. | Angle 2 theta (degrees) | Height (counts) | Area (counts x degrees) | FWHM (degrees) | Interplanar distance (A) |
|---|---|---|---|---|---|
| 1 | 4.1 | 1115 | 110 | 0.1004 | 21.753 |
| 2 | 6.8 | 183 | 145 | 0.8029 | 12.907 |
| 3 | 8.4 | 755 | 75 | 0.1004 | 10.531 |
| 4 | 10.9 | 1104 | 128 | 0.1171 | 8.087 |
| 5 | 12.2 | 195 | 19 | 0.1004 | 7.268 |
| 6 | 14.3 | 569 | 75 | 0.1338 | 6.214 |
| 7 | 14.7 | 1847 | 274 | 0.1506 | 6.013 |
| 8 | 15.3 | 1734 | 315 | 0.184 | 5.802 |
| 9 | 16.3 | 1164 | 154 | 0.1338 | 5.442 |
| 10 | 16.8 | 3420 | 734 | 0.2175 | 5.269 |
| 11 | 17.5 | 790 | 78 | 0.1004 | 5.069 |
| 12 | 17.9 | 33 89 | 503 | 0.1506 | 4.960 |
| 13 | 19.2 | 2467 | 407 | 0.1673 | 4.635 |
| 14 | 19.8 | 145 | 29 | 0.2007 | 4.477 |
| 15 | 20.4 | 313 | 52 | 0.1673 | 4.362 |
| 16 | 21.2 | 928 | 169 | 0.184 | 4.198 |
| 17 | 21.7 | 2093 | 414 | 0.2007 | 4.099 |
| 18 | 22.2 | 3850 | 635 | 0.1673 | 4.007 |
| 19 | 22.5 | 576 | 76 | 0.1338 | 3.948 |
| 20 | 23.1 | 1588 | 236 | 0.1506 | 3.855 |
| 21 | 24.8 | 1665 | 247 | 0.1506 | 3.594 |
| 22 | 25.2 | 1212 | 120 | 0.1004 | 3.534 |
| 23 | 25.6 | 1507 | 249 | 0.1673 | 3.477 |
| 24 | 26.7 | 2042 | 303 | 0.1506 | 3.342 |
| 25 | 27.6 | 2281 | 414 | 0.184 | 3.229 |
| 26 | 28.4 | 485 | 96 | 0.2007 | 3.138 |
| 27 | 29.6 | 599 | 99 | 0.1673 | 3.014 |

Measurement conditions have been disclosed in US 2007/0082885, art. [0016] et subseq.

It appears that the delta-form of ivabradine hydrochloride has been only obtained when acetonitrile has been used as the solvent for crystallization process. Apart of the direct disclosure in EP 1775288, the delta-form is also inherently obtainable by the process conditions disclosed in the Example 2 of the original EP 534859 as well as by similar process disclosed in more recent WO 2008/065681. Acetonitrile, however, is a classified (class 2) solvent in pharmaceutical industry that needs to be properly removed from any product that is intended to be formulated in pharmaceutical compositions.

It must be reminded that the delta-form of ivabradine hydrochloride is a hydrated form. Thus the crystallization of such form from acetonitrile is a complex process as water must be present as well. Either water is present in the starting material or water is present in the solvent (acetonitrile of reagent grade typically comprises less than 0.1 % of water) or, which was proven as an important aspect, water is provided by aerial humidity of the environment during isolation and storage. The thorough study of the process revealed that, while crystallization from acetonitrile as taught by the prior art may lead to form delta as the final product, initially a mixed hydrated/solvated intermediate comprising adsorbed or solvated acetonitrile was formed. When exposed to air, this mixed hydrate/solvate is converted into the desired fully hydrated state (isomorph), but quite slowly. In one example, it took up to 7 days to obtain fully hydrated form delta (i.e. without acetonitrile). When air-drying has been replaced by vacuum-drying, the dehydrated form delta-d or a mixture of forms delta and delta-d has been obtained. Upon exposure to air, rehydration takes place.

In summary, easy isomorphic transformation during the production and isolation conditions disclosed in the prior art may result in a mixture of hydrated and anhydrated polymorphic forms, which represents a disadvantage in the further use in pharmaceutical formulations. Furthermore, freshly prepared and isolated solids obtained after crystallization of ivabradine hydrochloride from acetonitrile comprise adsorbed or solvated acetonitrile, which must be fully removed as acetonitrile is a classified solvent, which is forbidden in pharmaceutical products. Removal of the acetonitrile by heating however leads to the parallel removal of water and to formation of unstable anhydrate; when trying to avoid such conversion, the acetonitrile-comprising product must be dried at ambient temperature for several days.

It was now found out with surprise that the desired delta- form of ivabradine hydrochloride may be formed by a crystallization of ivabradine hydrochloride from another solvent system, wherein such crystallization exhibits the advantage in that the form delta is formed without contamination with other crystalline forms and in that no solvated intermediate, from which the solvent would have been necessarily removed, is primarily formed. As a result, simple air drying at room temperature and ambient humidity is sufficient to obtain the delta-form. The solvent system of the present invention is also pharmaceutically more acceptable than acetonitrile.

The crystallization process of the present invention comprises crystallization of ivabradine hydrochloride from a mixture of an aliphatic alcohol of 1-4 carbon atoms and an aliphatic/alicyclic hydrocarbon of 5-8 carbon atoms. The "alcohol" may be a single aliphatic alcohol, for instance ethanol, but it may also be a mixture of at least two alcohols. The "hydrocarbon" may be a single aliphatic/alicyclic hydrocarbon, for instance n-pentane, n-hexane, n-heptane or cyclohexane, but it may be also a mixture of at least two hydrocarbons. The alcohol is a primary solvent, in which ivabradine hydrochloride may be dissolved easily, particularly at enhanced temperature. The hydrocarbon serves as an antisolvent, which effectively decreases and/or moderates the overall solubility of ivabradine hydrochloride after the solution has been obtained, so that it enhances the overall yield of the precipitated product, particularly at diminished temperature.

During or after the addition of the hydrocarbon into the crystallization system, initiation of the crystallization by seeding with minute amounts of crystals of the form delta may be performed.

In the preferred aspect, the alcohol is ethanol and the hydrocarbon is n-heptane.

In a more detail, the process of the present invention comprises, in essence, the following steps:

### a) Providing a solution of ivabradine hydrochloride in an aliphatic alcohol of 1-4 carbon atoms, preferably at a temperature of at least 50°C

Typically, ivabradine hydrochloride is combined with the sufficient amount of the alcohol and heated to the preselected temperature until essentially complete dissolution. The preselected temperature is advantageously a temperature of at least 50°C and includes the reflux temperature. The obtained solution is maintained for certain time to ensure that no seeds of another form of ivabradine hydrochloride are present. Alternately, a flash filtration of the solution may be performed to remove traces of foreign solid material.

The starting ivabradine hydrochloride may be any form of solid state of ivabradine hydrochloride, both crystalline and amorphous. Alternately, ivabradine hydrochloride may be directly formed in the alcohol solvent, for instance by a reaction of ivabradine base with gaseous hydrogen chloride or with the alcoholic solution of hydrogen chloride.

Typically, 1 gram of ivabradine hydrochloride is dissolved in about 5 ml of ethanol and the mixture is heated to reflux.

### b) Adding aliphatic/alicyclic hydrocarbon of 5-8 carbon atoms to the obtained solution.

The hydrocarbon is advantageously added dropwise or portionwise to the stirred solution to assure proper homogenization throughout the dilution. Opalescence (some oil formation) may occur during the process.

During and after the addition of the hydrocarbon, heating of the mixture is advantageously stopped and the system is allowed to cool spontaneously or with a certain cooling rate.

Typically, about 2 ml of n-heptane are added per each 1 ml of ethanol used.

### c) Optionally, seeding the obtained mixture with seed crystals of the delta-form of crystalline ivabradine hydrochloride

The seeding with minute amounts of seed crystals of the delta-form of ivabradine hydrochloride may be performed during or after the addition of the hydrocarbon.

### d) Stirring the obtained suspension

The mixture is generally stirred without heating for a time of at least 15 minutes, for to facilitate the crystallization as much complete as possible. Advantageously, the suspension may be additionally stirred at a temperature lower than ambient, for instance at a temperature from -15 to +15 °C, typically at a temperature of about 0°C.

### e) Filtration of the solid and drying the solid at room temperature

The solid may be filtered by any filtration / centrifugation process; advantageously, the solid product is washed with the hydrocarbon after filtration. Care is to be taken that drying is performed at aerial humidity preferably not exceeding 55%.

It was proven by NMR and XRPD analysis that the raw product analyzed immediately after isolation is the desired hydrated delta-form of ivabradine hydrochloride without being contaminated by other known forms or by a solvate comprising molecules of solvent. Without wishing to be bound by any theory, the inventor speculates that it is because sufficient amount of water for formation of hydrated crystals is inherently present in the alcohol solvent, particularly when the reagent-grade ethanol has been used, and also because the alcohol solvent has apparently very low tendency to form solvates with ivabradine hydrochloride.

The ivabradine hydrochloride product obtained by the process of the present invention may be formulated into pharmaceutical compositions, for instance to tablet compositions for oral administration, and may be used in medicine, for instance in a treatment of angina pectoris.

The invention will be further described with reference to the following non-limiting examples. The "delta-form" of ivabradine hydrochloride obtained by the present invention and described in the examples has the XRPD pattern substantially corresponding with that as disclosed in the cited prior art. The "delta-form" denomination of this particular polymorph has consistent meaning in the prior art and thus it can be regarded as an acknowledged name.

In particular, the XRPD pattern of the delta-form of ivabradine hydrochloride comprises the following characteristic signals : 14.7, 15.3, 16.9, 17.8, 19.0, 21.7, 22.2, 24.6, 26.5, 27.5 ±0.2 degrees 2theta, when measured at CuKα radiation with a wavelength of 1.54060 Å using a primary monochromator. Full XRPD pattern of the delta-form of ivabradine hydrochloride is shown in Fig.1.

### EXAMPLES

### Example 1

5.0 g of ivabradine hydrochloride was dissolved in 25 ml of ethanol at reflux, while stirring. Reflux was maintained for about 20 minutes. To the solution, stirred at reflux, 50 ml n-heptane was added dropwise. Meanwhile, the mixture was allowed to cool to room temperature. During addition, minor opalescence occurred. After about 3/4 of the n-heptane was added, a few mg of form delta was added as seeds. The mixture was stirred at room temperature for about 20 minutes, during which an opaque suspension was formed. Then, the suspension was stirred at 0°C for an additional 15 minutes. The mixture was filtered over a P3-glass filter (reduced pressure, gentle air-flow). The solid was air-dried at RT/∼40%RH in a desiccator containing a saturated sodium iodide solution in water. An off-white powder with a good flowability was obtained. The yield was 5 grams.
XRPD (Bruker-AXS D8 Vario diffractometer with Θ/2Θ geometry (reflection mode), equiped with a Vantec PSD detector).: form delta (Fig. 1)

### Example 2

100 g of ivabradine hydrochloride was dissolved in 500 ml of ethanol at reflux, while stirred mechanically at 100 rpm. Reflux was maintained for about 60 minutes. The mixture was allowed to cool down. After about 10 minutes, 1000 ml of n-heptane was added slowly in a 10-15 minute period, while mechanically stirred at 200 rpm. After addition of about 50% of the anti-solvent, about 1 gram of form delta was added as seeds. The mixture was further allowed to cool to about 20°C, while stirred at 200 rpm. A white suspension was formed.

After 20-30 minutes, the suspension was filtered over a P3-glass filter (reduced pressure). The solid was washed with n-heptane and vacuum-dried overnight at RT. A white to off-white powder was obtained.
XRPD: form delta

### Example 3

5 g of ivabradine hydrochloride was dissolved in 25 ml of ethanol at reflux, while stirring. Reflux was maintained for about 20 minutes. The solution was allowed to cool to luke warm. Then, 50 ml n-pentane was added dropwise as anti-solvent. After about 10 ml anti-solvent was added, a few mg of form delta was added as seeds. The mixture was stirred at RT for a few minutes. The mixture was filtered over a P3-glass filter (reduced pressure). The solid was washed with n-pentane and air-dried at RT/∼40%RH. A white powder with a good flowability was obtained.
XRPD: form delta

### REFERENCE EXAMPLES

### Reference example 1 (example 2 of EP 534,859, step E)

1.12 g of ivabradine free base was dissolved in 23.5 ml 0.1 M HCl. The solution was stirred, concentrated and the oily residue was dissolved in 8 ml of acetonitrile while heating. The clear solution was stored at room temperature for 2 hours, but no solid was formed. The flask was stored overnight at 4 °C. Again, no solid was formed. The solvent was partly evaporated and the residue was stored overnight at -20 °C. The solid was isolated, washed with a few ml of acetonitrile and air-dried at R.T. for about 1 hour.
TGA: About 8.3 m% below 185 °C, caused by evaporation of acetonitrile (cf. NMR)
XRPD: peak values similar to those of form delta as given in EP 1775288
NMR: ∼2.6 eq. acetonitrile

A part of the solid was left overweekend at R.T. under ambient conditions
TGA: About 3.0 m% below 130 °C, max. 0.9 eq. of water or 0.4 eq. acetonitrile
XRPD: peak values similar to those of form delta-d as given in US 7384932, tiny peaks of form delta.
NMR: ∼0.1 eq. acetonitrile, but some water is present

Another part was vacuum-dried overweekend at R.T.
TGA: About 3.0 m% below 125 °C (max. 0.6 eq. of water or 0.3 eq. acetonitrile) and 0.9 m% between 125-195 °C (max. 0.3 eq. water or 0.1 eq. acetonitrile)
XRPD: shows peaks of form delta and form delta-d
NMR: ∼0.33 eq. ACN, but some water is present

### Reference example 2 (EP 534859)

5.0 g of ivabradine hydrochloride was suspended in 80 ml of acetonitrile and heated to reflux, while stirring. No clear solution was obtained. Addition of 20 ml acetonitrile and reflux was necessary to obtain a clear solution. Reflux was maintained for 15-30 min. Then, the solution was allowed to cool to R.T. (solid formed) and stirred at R.T. for 30 min. To increase the yield, the suspension was stirred at 0°C for an additional 30 minutes. The suspension was filtered over a P3-glass filter (reduced pressure) and air-dried at R.T. for in total 7 days. A white to off-white powder was obtained.
NMR after 1 day: about 0.2 eq. acetonitrile was detected
NMR after 2 days: about 0.1 eq. acetonitrile was detected
NMR after 7 days: no acetonitrile was detected
TGA after 7 days: 1.9-2.2 eq. of water
XRPD after 7 days: form delta

### Reference example 3 (example 1 of US2007/0082886, ex. 1 of EP1775288)

About 80 ml of acetonitrile was heated to about 70°C, while stirring. To the hot solvent, 1 gram of ivabradine hydrochloride was added in portions. As a result, a clear solution was obtained. The solution was allowed to cool to R.T. (not stirred) and left at R.T. for about 2 days (already solid in first 30 min at R.T.). The suspension was filtered over a P3-glass filter (reduced pressure). A part of the solid was left at air/R.T. for 1 hour and then analysed. A white, fine powder with lumps was obtained.
TGA: About 7.85 m% below 190°C, caused by evaporation of acetonitrile and water (see NMR)
XRPD: form delta
NMR: maximally 1.5 eq. water and maximally 0.75 eq. acetonitrile

## Claims

1. A process of making the delta-form of crystalline ivabradine hydrochloride, comprising crystallization of ivabradine hydrochloride from a mixture of at least one aliphatic alcohol of 1-4 carbon atoms and at least one aliphatic/alicyclic hydrocarbon of 5-8 carbon atoms, said form being **characterized by** an x-ray powder diffraction pattern that includes characteristic peaks at the following 2 theta (± 0.2) angles: 14.7°, 15.3°, 16.9°, 17.8°, 19.0°, 21.7°, 22.2°, 24.6°, 26.5° and 27.5° when measured at Cu Kα radiation with a wavelength of 1.54060 Å using a primary monochromator.

2. A process according to claim 1 comprising the steps of
a) Providing a solution of ivabradine hydrochloride in at least one aliphatic alcohol of 1-4 carbon atoms, preferably at a temperature of at least 50°C;
b) Adding at least one aliphatic/alicyclic hydrocarbon of 5-8 carbon atoms to the obtained solution;
c) Optionally, seeding the obtained mixture with seed crystals of the delta-form of crystalline ivabradine hydrochloride;
d) Stirring the obtained suspension at ambient temperature; and
e) Filtration of the solid and drying the solid at room temperature and humidity.

3. The process according to claims 1-2, wherein the alcohol is ethanol and the hydrocarbon is n-pentane, n-hexane, n-heptane, cyclohexane or mixtures thereof.

## Patentansprüche

1. Verfahren zum Herstellen der delta-Form von kristallinem Ivabradin-Hydrochlorid, umfassend Kristallisieren von Ivabradin-Hydrochlorid aus einem Gemisch von mindestens einem aliphatischen Alkohol von 1-4 Kohlenstoffatomen und mindestens einem aliphatischen/alicyclischen Kohlenwasserstoff von 5-8 Kohlenstoffatomen, wobei die Form durch ein Röntgen-Pulverdiffraktionsmuster gekennzeichnet ist, das charakteristische Signalspitzen bei den folgenden 2 Theta (± 0,2) -Winkeln einschließt: 14,7°, 15,3° 16,9° 17,8°, 19,0° 21,7°, 22,2°, 24,6° 26,5°und 27,5°, wenn es bei Cu Kα-Strahlung mit einer Wellenlänge von 1,54060 Å unter Verwendung eines primären Monochromators gemessen wird.

2. Verfahren nach Anspruch 1, umfassend die Schritte
a) Bereitstellen einer Lösung von Ivabradin-Hydrochlorid in mindestens einem aliphatischen Alkohol von 1-4 Kohlenstoffatomen, bevorzugt bei einer Temperatur von mindestens 50°C;
b) Zufügen von mindestens einem aliphatischen/alicyclischen Kohlenwasserstoff von 5-8 Kohlenstoffatomen zur erhaltenen Lösung;
c) Gegebenenfalls Animpfen des erhaltenen Gemisches mit Impfkristallen der delta-Form von kristallinem Ivabradin-Hydrochlorid;
d) Rühren der erhaltenen Suspension bei Umgebungstemperatur; und
e) Filtration des Feststoffes und Trocknen des Feststoffes bei Raumtemperatur und Feuchtigkeit.

3. Verfahren nach Ansprüchen 1-2, wobei der Alkohol Ethanol ist, und der Kohlenwasserstoff n-Pentan, n-Hexan, n-Heptan, Cyclohexan oder Gemische davon ist.

## Revendications

1. Un procédé de fabrication de la forme delta cristalline du chlorhydrate d'ivabradine, comprenant la cristallisation du chlorhydrate d'ivabradine depuis un mélange formé d'au moins un alcool aliphatique comportant de 1 à 4 atomes de carbone et d'au moins un hydrocarbure aliphatique/alicyclique comportant de 5 à 8 atomes de carbone, ladite forme étant **caractérisée par** un spectre de diffraction aux rayons X sur poudre qui inclut des pics caractéristiques pour les angles 2-thêta (±°0,2) suivants: 14,7°, 15,3°, 16,9°, 17,8°, 19,0°, 21,7°, 22,2°, 24,6°, 26,5° et 27,5° lorsque la mesure est effectuée sous rayonnement Cu Kα présentant une longueur d'onde de 1,54060 Å avec un monochromateur primaire.

2. Un procédé selon la revendication 1, comprenant les étapes consistant à :
a) fournir une solution de chlorhydrate d'ivabradine dans au moins un alcool aliphatique comportant de 1 à 4 atomes de carbone, de préférence à une température d'au moins 50° C ;
b) ajouter à la solution obtenue, au moins un hydrocarbure aliphatique/alicyclique comportant de 5 à 8 atomes de carbone ;
c) ensemencer éventuellement, le mélange obtenu par des germes cristallins de la forme delta cristalline du chlorhydrate d'ivabradine ;
d) agiter à température ambiante la suspension obtenue ;
f) de séparation par filtration des solides et séchage à température ambiante et sous humidité.

3. Le procédé selon la revendication 1 - 2, dans lequel l'alcool est l'éthanol et l'hydrocarbure est le n-pentane, le n-hexane, le n-heptane, le cyclo-hexane ou leurs mélanges.
